(19) Europäisches Patentamt
European Patent Office
Office européen des brevets

(11) Veröffentlichungsnummer: **0 156 267**
**B1**

# (12) EUROPÄISCHE PATENTSCHRIFT

(45) Veröffentlichungstag der Patentschrift: **30.08.89**

(51) Int. Cl.$^4$: **C 07 H 17/04**

(21) Anmeldenummer: **85103024.7**

(22) Anmeldetag: **15.03.85**

## (54) Neues Verfahren zur Herstellung von Strictozidin.

(30) Priorität: **21.03.84 HU 111584**

(43) Veröffentlichungstag der Anmeldung:
**02.10.85 Patentblatt 85/40**

(45) Bekanntmachung des Hinweises auf die Patenterteilung: **30.08.89 Patentblatt 89/35**

(84) Benannte Vertragsstaaten:
**AT BE CH DE FR GB IT LI LU NL SE**

(56) Entgegenhaltungen:
**DE-A- 3 234 332**
**JOURNAL OF THE CHEMICAL SOCIETY (C), 1969, Seiten 1193-1200, The Chemical Society; A.R. BATTERSBY et al.: "Alkaloid Biosynthesis. Part XV.1 Partial Synthesis and Isolation of Vincoside and Isovincoside: Biosynthesis of the Three Major Classes of Indole Alkaloids from Vincoside"**

(73) Patentinhaber: **GYOGYNÖVENY KUTATO INTEZET**
**Jozsef A. u. 68**
**H-2011 Budakalász (HU)**

(72) Erfinder: **Kocsis, Ákos, Dr.**
**Kupecki u. 11**
**H-1035 Budapest (HU)**
Erfinder: **Pal, Zoltan, Dr.**
**Boroka u. 6**
**H-1025 Budapest (HU)**
Erfinder: **Szabo, Laszlo, Dr.**
**Budafoki u. 30**
**H-1111 Budapest (HU)**
Erfinder: **Tetenyi, Peter, Dr.**
**Népstadion u. 9**
**H-1143 Budapest (HU)**
Erfinder: **Varga geb. Balazs, Maria, Dr.**
**Jeszenak u. 98**
**H-1143 Budapest (HU)**

(74) Vertreter: **Hansen, Bernd, Dr.rer.nat. et al**
**Hoffmann, Eitle & Partner Patentanwälte Arabellastrasse 4 Postfach 81 04 20**
**D-8000 München 81 (DE)**

**EP 0 156 267 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein neues Verfahren zur Herstellung von Strictosidin der Formel (I).

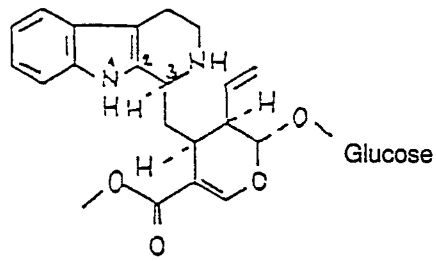

(I)

durch Kondensieren von Secologanin der Formel (II) mit Tryptamin der Formel (III) in wässriger Lösung.

Es ist bekannt, daß die Anzahl der Indolalkaloide heute schon tausend überschreitet, davon verfügen zahlreiche Verbindungen über biologische Aktivität. Neben Medikamentmolekülen großer Wirkung kommen einige Vertreter davon als halbsynthetischer Medikamentengrundstoff in Frage.

Die Alkaloide werden hauptsächlich mit drei Methoden hergestellt: durch Isolieren aus Pflanzen, durch halbsynthetische Umsetzung von natürlichen Grundstoffen bzw. durch Totalsynthese. Die halbsynthetische Methode ist von besonders großer Bedeutung, da die Prekursoren einiger Alkaloide großer Wirkung in viel größeren Mengen und billiger isoliert werden können als eventuell die der erwünschten Zielverbindung.

Eine Schlüsselverbindung der Biosynthese von Alkaloiden mit Indolgerüst ist das Strictosidin der Formel (I), früher unter dem Namen Isovincosid bekannt. Die biogenetische Rolle der Verbindung sowie die Möglichkeiten zur Herstellung von Alkaloiden unterschiedlicher Struktur werden in mehreren zusammenfassenden Artikeln ausführlich beschrieben. (R. T. Brown « Biomimetic Conversion of Secologanin into Alkaloids » Annual Proceedings of the Phytochemical Society of Europe. 1980. 17. 171-184).

Das Strictosidin kann auf zwei verschiedene Weisen hergestellt werden. Das eine ist die Isolation aus Pflanzen, z. B. aus Vinca rosea, das andere die partielle Synthese. Beide Methoden bringen zahlreiche Probleme mit sich.

Der Nachteil der Isolationsmethode besteht darin, daß die verwendeten Pflanzenarten das Strictosidin nur in sehr geringen Mengen enthalten, und das Trennen von den Partneralkaloiden das Anwenden dieser Methode sehr kompliziert und gleichzeitig kostspielig macht (siehe Journal of Chemical Society [C] 1969, 1193-99). Die technik wurde in den späteren Jahren geändert, aber dadurch wurden weder die Kostenaufwendungen noch die Menge des gewonnenen Stoffes wesentlich beeinflußt.

Die halbsynthetische Methode erwies sich schon als ein gangbarerer Weg zur Herstellung der Verbindung. Bei dem von Battersby et al. erarbeiteten Verfahren (J. Chem. Soc. [C] 1969. 1193-99) wird Secologanin der Formel (II)

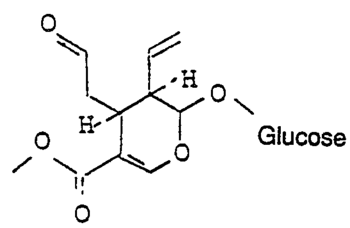

(II)

mit dem Hydrochloridsalz von Triptamin der Formel (III)

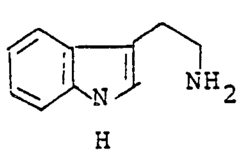

(III)

in einem Phosphatpuffer bei einer Temperatur von 37 °C 48 Stunden lang kondensiert. In der Reaktion entstehen zwei Produkte, das Strictosidin der Formel (I) und dessen 3β-Epimer, ähnlich wie Vincosid in Form eines Hydrochloridsalzes im Verhältnis von 1 : 1. Die zwei Verbindungen werden durch Chromatographie auf einer Cellulosesäule getrennt. Dieses Verfahren, das insgesamt auf die beiden Alkaloide gerechnet nur eine Ausbeute von 60 % aufweist, kann nur in Mikroausmassen eingesetzt werden. Bei größeren Mengen können die Isomere nur durch eine noch kompliziertere und deshalb kostspieligere Methode — durch Craig-Extraktion mit mehr als 1000 Schritten — getrennt werden, und die Gesamtausbeute sinkt auf 35 %.

Strictosidin-Hydrochlorid und Vincosid-Hydrochlorid können durch selektive Lactamisierung von Vincosid getrennt werden, z. B. wird die Lösung der beiden Hydrochloridsalze mit überschüssigem Natriumcarbonat versetzt, dann mit Säure behandelt, worauf Vincosidlactam ausgeschieden wird und Strictosidin-Hydrochlorid in der Lösung bleibt. In diesem Falle stört die beträchtliche Menge der gebildeten anorganischen Salze die Isolierung von Strictosidin-Hydrochlorid.

Diese Nachteile verhindern auch die Herstellung von entsprechenden Mengen Strictosidin in Laboratorien.

Der Erfindung liegt die Aufgabe zugrunde, für die Herstellung von Strictosidin ein leicht durchführbares und mit guter Ausbeute realisierbares Verfahren zu sichern.

Die Untersuchungen hatten zum Ziel, ähnlich wie die sich in den Pflanzen abspielende Biosynthese durch die Kondensation von Secologanin und Tryptamin Strictosidin herzustellen.

Aus der Fachliteratur ist bekannt, dass beide Verbindungen (Strictosidin und Vincosid) in Basenform zur Bildung von Lactam neigen. Diese Eigenschaft ist jedoch bei Vincosid viel ausgeprägter als bei Strictosidin (Annual Proceedings of the Phytochemical Society of Europe, 1980, 17, 172).

Die Lösung der der Erfindung zugrundeliegenden Aufgabe liegt in der überraschenden Erkenntnis, dass Strictosidin in Form eines Hydrochloridsalzes und Vincosid in Form von Lactam entsteht, wenn Tryptamin zum Teil als Hydrochloridsalz und zum Teil als freie Base verwendet wird. So wird das Isolieren sehr einfach, da das wasserunlösliche Vincosidlactam durch einfaches Filtern abgetrennt werden kann. Um das Auflösen der gesamten Menge der Tryptaminbase zu sichern, wird der wässrigen Lösung ein zusätzliches protisches Lösungsmittel, vorzugsweise Essigsäure, zugegeben, wobei die Menge jedoch höchstens 5 Gew.% des Reaktionsgemisches ausmacht.

Die Erfindung betrifft dementsprechend ein neues Verfahren zur Herstellung von Strictosidin durch das Umsetzen von Secologanin der Formel (II) und Tryptamin der Formel (III) in wässriger Lösung. Erfindungsgemäss wird das Tryptamin in Form eines Gemisches von freier Tryptaminbase und dem mit einer Mineralsäure, vorteilhaft Salzsäure, gebildeten Salz von Tryptamin in einem Molverhältnis zwischen 20 : 80 und 80 : 20, vorteilhaft 50 : 50, verwendet, und die Reaktion erfolgt vorteilhaft in Gegenwart eines zusätzlichen protischen Lösungsmittels, vorzugsweise Essigsäure, das in einer Menge zugegeben wird, die wenigstens das Auflösen der gesamten Menge der Tryptaminbase sichert, höchstens jedoch 5 Gew.% des Reaktionsgemisches ausmacht. Das entstehende Vincosidlactam der Formel (IV) kann durch Filtrieren abgeschieden und das Strictosidin in bekannter Weise isoliert werden.

Das Triptamin ist heutzutage auch schon im Handel leicht erhältlich, das Secologanin ist in einigen Pflanzen in hohem Anteil enthalten. Für seine Gewinnung auch in großen Mengen wird eine gut anwendbare Methode in dem Verfahren der ungarischen Patentanmeldung Nr. 1176/82 beschrieben.

Bei dem erfindungsgemäßen Verfahren werden Triptamin und Secologanin in einem Molverhältnis von 1 : 1 angewendet.

Bei dem verfahren wird die Temperatur zwischen 40 und 110 °C, vorteilhaft zwischen 80 und 100 °C gehalten, die Reaktion erfolgt in einer normalen oder einer inerten Atmosphäre, vorteilhaft in einer Stickstoffatmosphäre.

Das in unlöslicher Form abgeschiedene Vincosidlactam der Formel (IV)

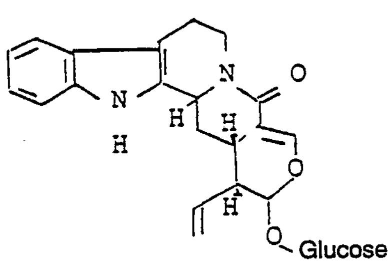

(IV)

wird am Ende der Reaktion durch Filtern entfernt und das Strictosidin wird nach dem Entfernen des Lösungsmittels bei vermindertem Druck auf bekannte Weise gewonnen.

Das erfindungsgemäße Verfahren, das leicht durchführbar und mit guter Ausbeute realisierbar ist, wird durch folgende Beispiele veranschaulicht.

Beispiel 1

0,16 g (0,1 mMol) Triptaminbase und 0,20 g (0,1 mMol) Triptaminhydrochlorid wurden im Gemisch von 10 ml Wasser und 0,5 ml Eisessig gelöst. Nach dem Auflösen wurden 0,82 g (0,2 mMol) Secologanin zugesetzt, und die Lösung wurde in einem $N_2$-Strom hoher Reinheit in einem Glyzerinbad 6 Stunden lang bei einer Temperatur von 100 °C gehalten. Nach einer Nacht Stehen wurde das abgeschiedene Vincosidlactam gefiltert und die Mutterlauge noch mit 3 × 30 ml Äthylacetat ausgeschüttelt. Die wäßrige Phase wurde bei vermindertem Druck zur Trockne eingedampft.

Gewicht des abgeschiedenen Vincosidlactams : 0,27 g.

$^1$H-nmr ($CD_3OD$) : 6,95-7,50 m, 5H Ar-H und C(17)-H ; 5,49 d, 1H C(19)-H ($J_0$ = 1,5 Hz) ; 4,74 d, 1H C(1')-Zucker-H ($J_0$ = 7,5 Hz) ; 3,30-3,41 ppm m, 4H C(5)-$H_2$ und C(6)-$H_2$.

Gewicht von Strictosidin : 0,67 g, Ausbeute : 62 %.

$^1$H-nmr ($CD_3OD$) in ppm-Werten : 7,76 s, 1H C(9)-H ; 6,90-7,45 m 4H Ar-H und C(17)-H ; 5,86 d, 1H C(19)-H ($J_0$ = 6 Hz) ; 3,81 s, 3H (CO)$OCH_3$ ; 4,84 d 1H C(1')-H-Zucker ($J_0$ = 7,5 Hz).

Gemeinsames Gewicht der zwei Alkaloide : 0,94 g. Ausbeute : 86 %.

Beispiel 2

0,096 g (0,6 mMol) Triptaminbase und 0,28 g (1,4 mMol) Triptaminhydrochlorid wurden im Gemisch von 10 ml Wasser und 0,3 ml Eisessig aufgelöst. Nach dem Auflösen wurden 0,82 g (2 mMol) Secologanin zugesetzt und die Lösung wurde auf Luft 6 Stunden lang Rücklauf unterworfen. Das Reaktionsgemisch wurde abgekühlt, eine Nacht lang stehengelassen, das abgeschiedene Vincosidlactam abgefiltert und die Mutterlauge mit 3 × 30 ml Äthylacetat ausgeschüttelt. Die wäßrige Phase wurde bei vermindertem Druck zur Trockne eingedampft.

Gewicht des abgeschiedenen Vincosidlactams : 0,23 g.
Gewicht von Strictosidin : 0,54 g, Ausbeute : 50 %.
Gemeinsames Gewicht der zwei Alkaloide : 0,77 g, Ausbeute : 72 %.

Beispiel 3

0,224 g (1,4 mMol) Triptaminbase und 0,12 g (0,6 mMol) Triptaminhydrochlorid wurden in einem Gemisch von 10 ml Wasser und 0,5 ml Eisessig gelöst, nach dem Auflösen wurden 0,82 g (2 mMol) Secologanin zugesetzt, und die Lösung wurde bei einer Temperatur von 100 °C 6 Stunden lang Rücklauf unterworfen. Das Reaktionsgemisch wurde abgekühlt, und nach einer Nacht Stehen das abgeschiedene Vincosidlactam abgefiltert. Die Mutterlauge wurde mit 3 × 30 ml Äthylacetat ausgeschüttelt und die wäßrige Phase bei vermindertem Druck eingedampft.

Gewicht des abgeschiedenen Vincosidlactams : 0,36 g.
Gewicht von Strictosidin : 0,40 g, Ausbeute : 37 %.
Gemeinsames Gewicht der zwei Alkaloide : 0,76 g, Ausbeute : 71 %.

Beispiel 4

0,16 g (1 mMol) Triptaminbase und 0,20 g (1 mMol) Triptaminhydrochlorid wurden in einem Gemisch von 10 ml Wasser und 0,5 ml Eisessig gelöst. Nach dem Auflösen wurden 0,82 g (2 mMol) Secologanin zugesetzt und die Lösung wurde in sehr reinem $N_2$-Strom auf einem Wasserbad bei einer Temperatur von 60 °C 14 Stunden lang gehalten. Das Gemisch wurde abgekühlt, dann nach einer Nacht Stehen das abgeschiedene Vincosidlactam abgefiltert, die Mutterlauge mit 3 × 30 ml Äthylacetat ausgeschüttelt. Die wäßrige Phase wurde bei vermindertem Druck zur Trockne eingedampft.

Gewicht des abgeschiedenen Vincosidlactams : 60 %.
Gewicht von Strictosidin : 0,635 g, Ausbeute : 60 %.
Gemeinsames Gewicht der zwei Alkaloide : 0,885 g, Ausbeute : 83 %.

## Patentansprüche

1. Verfahren zur Herstellung von Strictosidin der Formel (I)

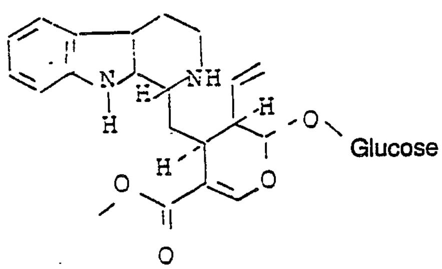

(I)

durch Kondensieren von Secologanin der Formel (II)

(Siehe Formel Seite 5 f.)

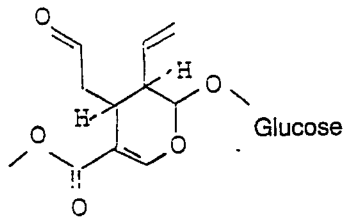

(II)

und Tryptamin der Formel (III)

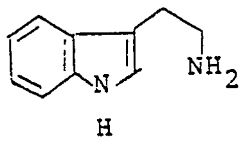

(III)

in einer wässrigen Lösung, dadurch gekennzeichnet, daß Tryptamin in einem Molverhältnis von (20-80) : (80-20), vorteilhaft in einem Molverhältnis von 50 : 50, als Gemisch der freien Tryptaminbase und eines mit einer Mineralsäure gebildeten Salzes von Tryptamin eingesetzt wird, und daß der wässrigen Lösung ein zusätzliches protisches Lösungsmittel, vorzugsweise Essigsäure, in einer Menge zugegeben wird, die wenigstens das Auflösen der gesamten Menge der Tryptaminbase sichert, höchstens jedoch 5 Gew.% des Reaktionsgemisches ausmacht, und das entstehende Vincosidlactam der Formel (IV)

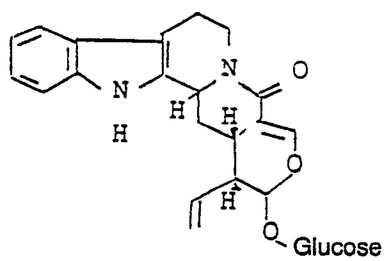

(IV)

durch Filtrieren abgeschieden und das Strictosidin in bekannter Weise isoliert wird.

2. Verfahren nach Anspruch 1, dadurch gekennzeichnet, dass als mit einer Mineralsäure gebildetes Salz von Tryptamin sein mit Schwefelsäure oder Salzsäure gebildetes Salz, vorteilhaft Tryptaminhydrochlorid, verwendet wird.

3. Verfahren nach Anspruch 1, dadurch gekennzeichnet, dass die Reaktion bei einer Temperatur zwischen 40 und 110 °C, vorteilhaft zwischen 80 und 100 °C, erfolgt.

4. Verfahren nach Anspruch 1, dadurch gekennzeichnet, dass die Reaktion in einer normalen oder inerten Atmosphäre, vorteilhaft in einer Stickstoffatomosphäre, durchgeführt wird.

## Claims

1. Method for the preparation of strictosidine of the formula (I)

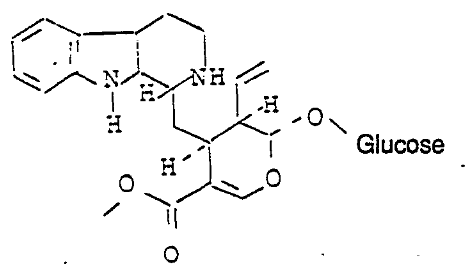

(I)

by condensing secologanin of formula (II)

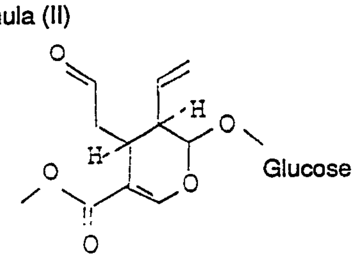

(II)

and tryptamine of formula (III)

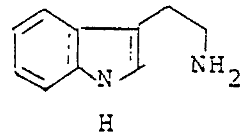

(III)

in an aqueous solution, characterised in that the tryptamine is used in a molar ratio of (20-80) : (80-20), advantageously in a molar ratio of 50 : 50, as a mixture of the free tryptamine base and a salt of tryptamine formed with a mineral acid, and to the aqueous solution is added an additional protonic solvent, preferably acetic acid, in a quantity which at least ensures dissolution of the whole quantity of the tryptamine base, but comprises not more than 5 wt.% of the reaction mixture, and the resulting vincoside lactam of formula (IV)

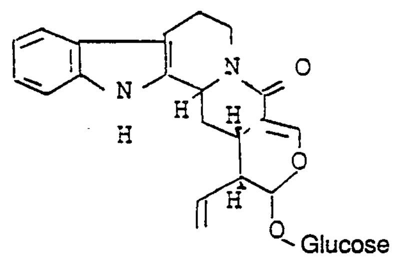

(IV)

is precipitated by filtration, and the strictosidine isolated in a known manner.

2. Method according to claim 1, characterised in that as the salt of tryptamine formed with a mineral acid there is used the salt thereof formed with sulphuric acid or hydrochloric acid, advantageously tryptamine hydrochloride.

3. Method according to claim 1, characterised in that the reaction takes place at a temperature between 40 and 110 °C, advantageously between 80 and 100 °C.

4. Method according to claim 1, characterised in that the reaction is carried out in a normal or an inert atmosphere, advantageously in a nitrogen atmosphere.

## Revendications

1. Procédé de préparation de la strictosidine de formule (I)

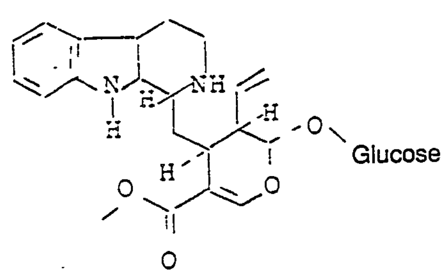

(I)

par condensation de la sécologanine de formule (II)

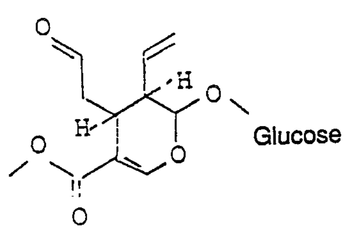

(II)

et de la tryptamine de formule (III)

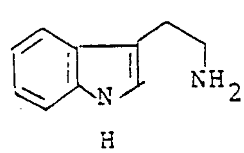

(III)

en solution aqueuse, caractérisé en ce que l'on introduit la tryptamine sous forme d'un mélange de tryptamine base libre et d'un sel de la tryptamine avec un acide minéral dans un rapport molaire de 20/80 à 80/20, de préférence dans un rapport molaire de 50/50, en ce que l'on ajoute à la solution aqueuse un solvant protique supplémentaire, de préférence l'acide acétique, en une quantité qui assure au moins la dissolution de la quantité totale de tryptamine base et qui toutefois constitue au maximum 5 % en poids du mélange réactionnel, en ce que l'on sépare par filtration la vincosidelactame de formule (IV)

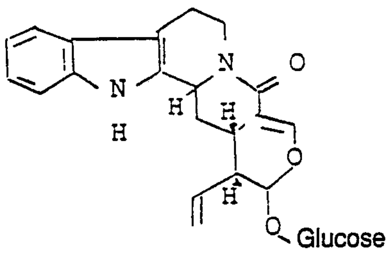

(IV)

formée et isole la strictosidine par une méthode connue.

2. Procédé selon la revendication 1, caractérisé en ce que l'on utilise, en tant que sel de la tryptamine avec un acide minéral, le sel formé par la tryptamine avec l'acide sulfurique ou l'acide chlorhydrique, de préférence le chlorhydrate de tryptamine.

3. Procédé selon la revendication 1, caractérisé en ce que l'on effectue la réaction à une température située entre 40 et 110 °C, de préférence entre 80 et 100 °C.

4. Procédé selon la revendication 1, caractérisé en ce que l'on effectue la réaction sous une atmosphère normale ou inerte, de préférence sous une atmosphère d'azote.